Europäisches Patentamt

European Patent Office

Office européen des brevets

(19)

(11) Publication number: **0 456 307 A1**

(12) **EUROPEAN PATENT APPLICATION**

(21) Application number: 91201042.8

(22) Date of filing: 02.05.91

(51) Int. Cl.⁵: **G01N 33/571**, G01N 33/563,
G01N 33/569

(30) Priority: 11.05.90 US 522444

(43) Date of publication of application:
13.11.91 Bulletin 91/46

(84) Designated Contracting States:
AT BE CH DE DK FR GB IT LI LU NL SE

(71) Applicant: **EASTMAN KODAK COMPANY**
343 State Street
Rochester, New York 14650-2201(US)

(72) Inventor: **Mauck, John Charles, c/o Eastman
Kodak Company**
Patent Department, 343 State Street
Rochester, New York 14650-2201(US)
Inventor: **Boyer, Bradley Porter, c/o Eastman
Kodak Company**
Patent Department, 343 State Street
Rochester, New York 14650-2201(US)
Inventor: **Warren, Harold Chester, c/o
Eastman Kodak Company**
Patent Department, 343 State Street
Rochester, New York 14650-2201(US)
Inventor: **Sprague, Lisa Dumers, c/o Eastman
Kodak Company**
Patent Department, 343 State Street
Rochester, New York 14650-2201(US)
Inventor: **Snodgrass, Gary Louis, c/o Eastman
Kodak Company**
Patent Department, 343 State Street
Rochester, New York 14650-2201(US)

(74) Representative: **Baron, Paul Alexander
Clifford et al**
Kodak Limited Patent Department Headstone
Drive
Harrow Middlesex HA1 4TY(GB)

(54) Use of enzyme-labeled antibody fragment in determination of chlamydial or gonococcal antigens.

(57) An enzyme-labeled F(ab')₂ fragment is useful in the determination of Chlamydial or Gonococcal organisms or antigens thereof. The fragment is used in place of labeled whole antibodies to form an immunological complex bound to a microporous membrane. This complex is readily detected using appropriate dye-providing compositions composed of a substrate for the enzyme label. The labeled antibody fragment can be provided as a buffered composition in a diagnostic test kit.

The present invention relates to a diagnostic test kit and a buffered composition comprising an enzyme-labeled F(ab')₂ fragment directed to chlamydial or gonococcal antigens. It also relates to a rapid and accurate method for detection of such antigens using the buffered composition.

Immunoassays have been used in recent years to detect the presence or amount of infectious agents in animals or humans. In order for the assay to be useful, it must detect a particular infectious agent with a high degree of reliability. In most cases, this requires the isolation and reaction of antigens peculiar to the organism with corresponding antibodies. For the test to be commercially successful, it also needs to be relatively inexpensive, simple to use and rapid.

One such organism which can be detected by immunoassays is Chlamydia trachomatis (herein C. trachmatis) which is one of two microbial species of the genus Chlamydiaceae, order Chlamydiales. There are 15 or more strains of this species which are the causes of a number of human ocular and genital diseases including trachoma, inclusion conjunctivitis, lymphogranuloma venereum, nongonococcal urethritis and proctitis. Infection from C. trachomatis is pervasive in the general population so that it is believed that there are millions of cases each year of nongonococcal urethritis alone.

Gonorrhea is a disease usually transmitted by sexual contact caused by a bacterium of the Neisseria genus, especially N. gonorrhoeae. The disease has plagued mankind for thousands of years, and although antibiotics have helped control its spread, it still persists in epidemic proportions in many parts of the world. The importance of detection and treatment of this organism is well recognized. N. meningitidis and N. lactamica are also species of considerable medical and diagnostic interest.

Because of the widespread nature of these diseases, there is considerable interest in having a rapid, simple and reliable test for detection of chlamydial and gonococcal organisms. Considerable research has been carried out to find useful ways to extract and detect antigens from chlamydial organisms. See, for example, US-A-4,427,782 and US-A-4,663,291 and EP-A-0 174 106 and EP-A-0 193 431.

Assays for C. Trachomatis and N. gonorrhoeae carried out using a solid support are described in US-A-4,497,899 and US-A-4,497,900, respectively. The described assays are performed by extracting antigen from the organism and coating it on a bare solid support. The coated antigen is then detected with either one or two antibodies, one of which is suitably labeled. The critical feature of the assays appears to be the use of a solid support for attachment which is untreated or uncoated with any biological material. Attachment of antigen is apparently achieved by incubating the coated support for an extended time sufficient to cause adsorption of antigen thereon. The absorption time is at least 30 minutes at elevated temperature (37°C). The entire assay described in US-A-4,497,899 takes at least 3 hours to perform. A similar assay is described in US-A-4,497,900 for N. gonorrhoeae.

Other assays for chlamydial and gonococcal antigens are described in EP-A-0 325 045 but the length of time for the assay is up to 90 minutes at high temperatures (37°C). This assay utilizes indistinguishably either whole antibody molecules or fragments thereof.

It would be desirable to have a much more rapid test for chlamydial or gonococcal organisms which has high reliability and can be performed at room temperature.

Such an improvement is described and claimed in EP-A-0 363 109. It was found that ionically charged supports attract chlamydial or gonococcal antigen and enable one to quickly and sensitively detect such antigens. However, further improvements were needed for some biological specimens, especially those containing copious amounts of whole blood, mucus or components. Thus, the improvement described in EP-A-0 363 090 was made.

Despite the considerable improvements described in the copending applications noted above, there is a continued need to make the assay faster (that is, less than 20 minutes). It would be desirable to eliminate as many steps as possible from the earlier assay protocols so users would find the assays more convenient and suitable for prompt diagnosis and treatment of chlamydial and gonococcal infections. Moreover, the peroxidase-labeled antibody conjugate used in the assay has a certain degree of stability (and thus, shelf life), but there is a need for additional stability and longer shelf life.

The problems with known assays are overcome with a method for the determination of a chlamydial or gonococcal antigen comprising:

A. contacting chlamydial or gonococcal antigen from a specimen suspected of containing chlamydial or gonococcal organisms, respectively, with a microporous membrane which is substantially free of any biological compound reactive with the antigen so as to bind the antigen directly to the membrane,

B. within 5 minutes of the contact in step A, contacting the bound antigen with an immunological composition comprising: an enzyme-labeled F(ab')₂ fragment directed to the chlamydial or gonococcal antigen, respectively,

so as to form a labeled immunological complex bound to the membrane, and

C. within 10 minutes of the contact in step B, determining the presence or amount of the bound labeled

2

complex on the membrane as a measure of the presence or amount of chlamydial or gonococcal organisms, respectively, in the specimen.

This invention also provides a buffered immunological composition comprising an enzyme-labeled immunoreactant, the composition characterized wherein the immunoreactant is a F(ab')$_2$ fragment directed to either a chlamydial or gonococcal organism or to an antigenic component thereof.

This invention provides for the use of a buffered immunological composition comprising an enzyme-labeled F(ab')$_2$ fragment directed to either a chlamydial or gonococcal organism or to an antigenic component thereof.

Further, a diagnostic test kit useful for the determination of chlamydial or gonococcal organisms comprises:

A. a buffered immunological composition comprising an enzyme-labeled immunoreactant, and

B. a dye-providing composition comprising a substrate for the enzyme label,

the test kit characterized wherein the immunoreactant is a F(ab')$_2$ fragment directed to either a chlamydial or gonococcal organism or to an antigenic component thereof.

Further, a method for the determination of a chlamydial or gonococcal antigen comprises:

detecting the presence or amount of either an uncomplexed enyzme-labeled F(ab')$_2$ fragment directed to a chlamydial or gonococcal antigen or an enzyme-labeled complex of the enzyme-labeled fragment formed with the antigen, after the antigen is contacted with a buffered composition comprising the enzyme-labeled fragment, and after uncomplexed fragment is separated from any complex formed between the antigen and enzyme-labeled fragment.

Also, this invention provides an enzyme immunoassay employing F(ab')$_2$ antibody fragments conjugated with an enzyme to form enzyme conjugates for detecting the presence or amount of a chlamydial or gonococcal organism in a specimen,

the immunoassay comprising addition of a buffered composition comprising a conjugate of an enzyme and an F(ab')$_2$ fragment which is specifically reactive with a chlamydial or gonococcal organism, or with an antigenic component thereof.

The method of this invention is very rapid in the detection of chlamydial or gonococcal organisms, requiring less than 15 minutes, and particularly rapid after extraction of antigen occurs, that is requiring less than 10 minutes. The assay is also reliable in providing accurate results in the presence of multiple chlamydial or gonococcal strains, and is simple to use. It is particularly useful for the detection of the lipopolysaccharide antigen of Chlamydia trachomatis (C. Trachomatis). However, it is also useful for rapid detection of proteins IA and IB from Neisseria gonorrhoeae (N. gonorrhoeae).

Moreover, the invention has improved shelf life because the labeled immunoreagent is more stable. This is achieved by using a labeled F(ab')$_2$ fragment instead of a labeled whole antibody in the immunoassay. The fragment is directed to the chlamydial or gonococcal organism or to an antigenic component thereof. This fragment is conjugated to an enzyme label, and thus only a single immunoreagent is needed in the assay. In many known assays, at least two antibodies are used, for example in the preferred embodiments of EP-A-0 363 090.

The present invention comprises a method for determining the presence of C. trachomatis (or other chlamydial species), or the presence of N. gonorrhoeae (or other gonococcal species) in a biological specimen which has been obtained from a patient using any suitable medical or diagnostic techniques. Such specimens include, for example, swab specimens obtained from the cervix, urethra, throat or anus of a patient, and body fluids such as synovial fluid or fluid from lesions. The biological specimens so obtained are suspected of containing bacterial organisms which comprise the chlamydial or gonococcal antigen (or mixture thereof) to be determined.

While the assay can be carried out to detect antigenic sites on intact chlamydial or gonococcal organisms, it is usually desirable to extract antigenic components (for example, lipopolysaccharides or proteins) from the organisms in order to increase assay sensitivity. Standard techniques can be used for lysing the organism to release antigen including, for example, solvent dilution or high pH lysing solutions, enzyme treatment, heating and physical agitation such as sonication or centrifugation. The use of anionic detergents or salts such as sodium dodecyl sulfate and deoxycholate is described in US-A-4,497,899, US-A-4,497,900 and US-A-4,663,291.

In a preferred embodiment, the present invention can be used to detect the chlamydial lipopolysaccharide (glycolipid group) antigen (which is described, for example, in EP-A-0 193 431). In another embodiment, the detected antigen can be the chlamydial major outer membrane protein of the organism which comprises 60% of the total associated outer membrane protein. This antigen and methods of extraction are described in US-A-4,427,782. In some instances, a mixture of these chlamydial antigens will be detected using a mixture of immunoreagents in the practice of the present invention. In still other embodiments, the invention is used to detect one or more gonococcal antigens (IA or IB protein), or mixtures of antigens from one or more gonococcal strains.

A preferred extraction composition includes an alcoholamine or salt thereof and has high pH. Further details of this preferred extraction composition are provided below in Example 2.

In addition, it may be desirable to use a protease in the extraction procedure to break down whole blood and mucus. This is demonstrated in Example 2 below.

Once antigen is extracted from the organism, it is desirable, although not essential, that the specimen be prefiltered to remove cell debris, particulate matter and other unwanted materials prior to further handling. Prefiltering can be carried out in a suitable container having a filter of some type.

Extraction can be carried out in any suitable container, including devices specially designed for extraction of antigen. Useful devices are known in the art, including US-A-4,746,614.

Extracted antigen is contacted with a microporous membrane generally having an average pore size of from 1 to 10 $\mu$meter, and preferably of 5 $\mu$meter. The membrane can be prepared from any suitable material that will maintain its integrity during the assay, including but not limited to, sintered glass, porous cellulosic materials, porous polymeric films and filter materials, woven fibers, and others known in the art. Preferably, the membrane is prepared from a polyamide, that is a long-chain synthetic polymer having recurring amide groups in the polymer backbone. They are generally copolymers of a diamine and a dicarboxylic acid, or homopolymers of a lactam of an amino acid. Representative materials include, but are not limited to, polyhexamethylene dodecanediamide (nylon 612), polyhexamethylene adipamide (nylon 66), poly-$\epsilon$-caprolactam (nylon 6), polyhexamethylene sebacamide (nylon 610) and poly-7-aminoheptaneamide (nylon 7), and mixtures thereof. Polyhexamethylene adipamide (nylon 66) is preferred. The membranes are preferably nonionic, although membranes having ionic charges can also be used, if desired. It is essential, however, that the membranes be substantially free of any biological compound reactive with the chlamydial or gonococcal antigen.

Further details of useful membrane materials and details of their preparation are found in various published sources including US-A-4,340,479 and Pall Corp. trade literature brochures PSD-750a (March, 1983, pp. 1-20) and NM-900c (September, 1984, pp. 1-28). Useful membranes are also described in EP-A-0 363 090 and EP-A-0 363 109. A preferred polyamide microporous membrane is the nylon 66 membrane manufactured and sold by Pall Corp. as Biodyne™ A or LoProdyne™ membranes.

In the practice of this invention, the membrane optionally can be coated or treated with one or more surfactants in an amount of at least 20 mg/m². Useful surfactants include, but are not limited to, anionic, amphoteric or nonionic surfactants, with nonionic surfactants being preferred. There are many useful surfactants, and a worker skilled in the art can consult the standard resource, McCutcheon's Emulsifiers and Detergents, 1986 Ed., McCutcheon Division Publishing Co., Glen Rock, N.J. to find useful surfactants. For example, useful anionic surfactants include, but are not limited to sodium dodecyl sulfate, lithium decyl sulfate, ammonium dodecyl sulfate, sodium decyl sulfate and others known in the art. Useful amphoteric surfactants include Zonyl™ FSK.

Particularly useful nonionic surfactants include fluorinated nonionic surfactants such as perfluoroalkylpoly(ethylene oxide) alcohols, for example commercially available as Zonyl™ FSN (DuPont), Nonidet™ P-40 (Sigma Chemical) or as Fluowet™ OT (American Hoechst).

The microporous membrane described herein can be used in combination with other equipment (bottles, test tubes, swabs, beakers or cups) in order to carry out the assay. Alternatively and preferably, it is fitted into a disposable test device in which the assay can be carried out and all fluids accommodated. Useful configurations of test devices are known in the art including US-A-3,825,410, US-A-3,888,629, US-A-3,970,429 and US-A-4,446,232. Particularly useful devices are described in US-A-4,833,087 and EP-A-0 308 231.

In the following description of a method of this invention, the procedure described is considered preferred. Other optional methods are detailed below.

Almost immediately upon contact of the antigen with the microporous membrane, the antigen is bound thereto. The antigen is preferentially bound to the membrane as opposed to other proteins, cell components, whole blood or mucus or other debris which may be present in the test specimen or reagents used in the assay.

Within 5 minutes, and preferably within 1 to 5 minutes, of the contact, unbound materials are separated from the bound antigen, and the bound antigen is contacted with the immunological composition of this invention so as to form an immunological complex bound directly to the support. Fluid and unbound materials may be removed prior to or simultaneously with this contact. A separate wash solution (described below) may be used if desired. If the assay is carried out using a disposable test device, fluid and unbound materials (such as whole blood and mucus components) in the specimen are allowed to flow through the membrane and collected in a suitable compartment during the time the antigen is bound to the membrane.

Whole antibody molecules can be broken down into various fragments depending upon the reagent used for the digestion. The whole molecule is composed of what are known as Fc (or fraction crystallizable) regions and Fab (or fraction antibody binding) regions. Digestion with pepsin or papain or other similar digestive enzyme cleaves the monovalent Fab' and divalent (Fab')$_2$ fragments from Fc fragment. Where only the divalent fragment is desired, purification techniques are available to separate it from the whole antibody, such as by chromatography.

The antibody fragment used in this assay is specifically immunoreactive with one or more chlamydial or gonococcal strains (depending upon what organism is of interest). The composition of this invention can include a plurality of F(ab')$_2$ fragments, each directed to one or more antigens of one or more strains of the organism of interest. The fragments can be obtained from either polyclonal or monoclonal antibodies. Polyclonal antibodies are commercially available or prepared in various animals using known techniques employing an antigen common to the strain of organism to be detected. Preferably, the antibodies used to prepare the desired fragments are monoclonal and are either commercially available or prepared using standard hybridoma technology. Useful procedures for preparing antibodies are described, for example, in EP-A-0 193 431 and US-A-4,427,782.

The F(ab')$_2$ fragments useful in this invention can be prepared using standard pepsin digestion to remove the Fc portions of the whole antibody molecules, as noted for example in US-A-4,397,960 and US-A-4,814,433. Digestion with deactivated papain may also be useful, according to Parkam and others, J. Immunol. Methods, 53, 133, (1982) and US-A-4,814,433.

Once fragments are formed, the F(ab')$_2$ fragment must be isolated from the other fragments. Chromatography can be used for this although other techniques are known. Preferably, the whole chlamydial or gonococcal antibody is treated with pepsin to cleave the molecule into F(ab')$_2$ and Fab' fragments. The resulting mixture is then purified in a suitable manner, for example, by chromatography and dialysis to obtain purified F(ab')$_2$.

The fragments can be labeled with an appropriate enzyme using known procedures, as described for example in US-A-4,361,647, US-A-4,810,638, J. Immunol., 109, pp. 129-35, 1972 (one step glutaraldehyde method). Another procedure is described by Imagawa and others in J.Appl.Biochem., 4, 51-57 (1982).

Useful enzyme labels include, but are not limited to, peroxidase, alkaline phosphatase, acid phosphatase, $\beta$-galactosidase, glucoamylase, glucose oxidase, acetylcholineesterase, catalase, lysozyme, malate dehydrogenase, glucose-6-phosphate dehydrogenase and $\beta$-amylase. Peroxidase and alkaline phosphatase are preferred with peroxidase being most preferred.

In a preferred method for labeling the F(ab')$_2$ fragment, the fragment is reacted with succinimidyl-4-(N-maleimidomethyl)cyclohexane-1-carboxylate to provide a F(ab')$_2$ fragment reagent. An enzyme, for example peroxidase, is reacted with S-acetylmercaptosuccinic anhydride to provide an enzyme reagent containing a reactive mercapto moiety. This moiety is then reacted with the F(ab')$_2$ reagent to provide the desired enzyme-labeled immunoreagent for use in the assay of this invention. Further details of this procedure are provided in Example 1 below.

The chlamydial or gonococcal antibody fragment can be supplied for the assay in an immunological composition further comprising one or more nonimmunological proteins which reduce nonspecific interactions on the membrane. By "nonimmunological protein" is meant proteins which do not bind in an immunological reaction with the antigen of interest to an appreciable extent. Useful nonimmunological proteins are well known and include, for example, casein, $\alpha$-casein, fetal bovine serum and porcine gamma globulin. Particularly useful components of such compositions include the protein and an amphoteric surfactant, as described in EP-A-0 363 091. The immunological composition is generally buffered to a pH of from 6 to 8 using suitable buffers known in the art, including but not limited to, phosphates, borates, phosphate buffered saline solution, tris(hydroxymethyl)aminomethane,
N-tris(hydroxymethyl)methyl-3-aminopropanesulfonic acid and
N-tris(hydroxymethyl)methyl-2-aminoethanesulfonic acid.

Once the bound antigen has been contacted with the immunological composition, a bound immunological complex is formed on the membrane almost immediately. To hasten the formation of this complex, the membrane and reagents can be incubated at a temperature of from 15 to 30°C for up to 10 minutes. Preferably, the incubation is carried out at from 18 to 25°C (that is, room temperature) for from 1 to 3 minutes. These mild incubation conditions are in sharp contrast to the 30 minutes at 37°C described as necessary for adsorption of chlamydial antigen to bare supports in US-A-4,497,899.

After this incubation and within 10 minutes (preferably within 1 to 3 minutes) of the beginning of complex formation, the bound complex is washed one or more times with a wash solution which generally has a pH of from 7 to 12. Such a wash solution can be used one or more times at any time in the assay.

The solution preferably contains one or more surfactants to aid in separating unbound materials from the bound complex. Particularly useful surfactants are cationic surfactants, as described in EP-A-0 363 108.

After the washing, the bound labeled complex is appropriately detected. Generally, in order to detect the bound labeled complex on the microporous membrane, an appropriate dye-providing composition is contacted with the complex. This composition contains a substrate for the enzyme so the enzyme directly or indirectly provides a detectable dye if the enzyme is present. The dye can be a single compound which is activated by enzymatic action, or it can be formed from the enzymatic action of two or more compounds, or from an intermediate formed from two or more compounds. Appropriate substrates would be readily apparent to one of ordinary skill in the art.

In a particularly preferred embodiment, the enzyme label is peroxidase, and at some point in the assay, hydrogen peroxide and a suitable dye-providing composition is added to provide a detectable dye. For example, useful dye-providing reagents include leuco dyes, such as triarylimidazole leuco dyes (as described in US-A-4,089,747, or other compounds which react to provide a dye in the presence of peroxidase and hydrogen peroxide (that is, compounds which react to provide a dye upon catalytic action of peroxidase).

Addition of the dye-providing composition is done relatively quickly after washing the bound complex, that is generally within 2 minutes, and preferably immediately thereafter. If desired, dye detection can be hastened with incubation if the reagents warrant it. The resulting dye is then detected using standard equipment and procedures to make visual or spectrophotometric readings.

A preferred method for the determination of a chlamydial antigen comprises:

A. extracting chlamydial antigen from chlamydial cells present in a biological specimen,

B. contacting the extracted chlamydial antigen with a polyamide microporous membrane which is substantially free of any biological compound reactive with the antigen so as to bind the antigen directly to the membrane,

C. within 2 minutes of the contact in step A, separating unbound materials from bound antigen, and contacting bound antigen with an immunological composition comprising: an enzyme-labeled $F(ab')_2$ fragment immunologically directed to the chlamydial antigen,

so as to form a labeled immunological complex bound to the membrane, and

D. separating uncomplexed materials from the bound labeled complex by washing, and

E. contacting the complex with a dye-providing composition which comprises a substrate for the enzyme label thereby determining the presence of the bound labeled complex on the membrane as a measure of the presence or amount of chlamydial organisms in the specimen,

the method from steps B to E being carried out in less than ten minutes.

The diagnostic test kit of the present invention comprises the immunological composition described herein and one or more other component compositions, solutions or devices for carrying out the assay. For instance, it generally includes the immunological composition comprising the labeled $F(ab')_2$ fragment, and an appropriate dye-providing composition in a separate container. Optional components of the kit include wash solutions, extraction compositions, extraction devices, swabs or other specimen collecting means, disposable test devices (including a microporous membrane fitted therein) and others known to one skilled in the art, all in appropriate packages or containers.

The following examples are provided to illustrate, but not limit the scope of, the present invention. All percentages are based on weight unless otherwise indicated.

Example 1: Preparation of Immunological Composition

This example illustrates the preparation of an immunological composition of this invention containing a $F(ab')_2$ antibody fragment directed to the lipopolysaccharide antigen of C. trachomatis.

Materials:

The antibodies used in preparing the $F(ab')_2$ anti-chlamydial fragment were 10G9 monoclonal antibodies directed to the chlamydial lipopolysaccharide antigen obtained from Cetus Corp. (Emeryville, California) in a phosphate buffered saline solution (6.96 mg/ml, pH 7.2).

Pepsin was obtained from Sigma Chemical Co. (catalog no. 6887, 3900 units/mg of protein).

Preparation of Fragment:

A phosphate buffered saline solution (14 ml) of the anti-chlamydial antibody (6.96 mg/ml) was dialyzed using an Amicon concentrator with a 30,000 molecular weight cutoff membrane and citrate buffer (56 ml, 0.1 molar, pH 4.1). The final volume was 9 ml containing 10.8 mg/ml of citrate buffer. This solution was mixed with pepsin (1.169 ml of a solution containing 2.5 mg/ml of citrate buffer), and the resulting mixture was rotated for two hours at 37°C. The pH was raised to 7 with tris(hydroxymethyl)aminomethane buffer (1 molar, pH 10.8), and the solution was dialyzed using an Amicon concentrator with a 30,000 molecular weight cutoff membrane using tris(hydroxymethyl)aminomethane buffer (20 mmolar, pH 8). The resulting solution (about 30 mL) was stored at 4°C until purified.

This product was purified on a DEAE-Sepharose column using first tris(hydroxymethyl)aminomethane buffer (25-30 ml, 20 mmolar, pH 8), then with the buffer (25 ml) and sodium chloride (0.75 molar). The portions containing the antibody fragments ($OD_{280}$) were pooled and dialyzed against 2-(N-morpholino)-ethanesulfonic acid (25 mmolar, pH 6.5) to give 21.8 mg total of $F(ab')_2$ and $F(ab')$ fragments.

These fragments were separated by chromatography using an ABx column and eluting with 2-(N-morpholino)ethanesulfonic acid (30 ml, 25 mMolar, pH 5.6), followed by a solution (30 ml) consisting of ammonium sulfate (0.5 molar) and sodium acetate (0.02 molar). The fractions containing the $F(ab')_2$ fragment were pooled, combined and dialyzed against phosphate buffered saline solution in an Amicon concentrator to give pure $F(ab')_2$ fragment (13.85 mg).

Preparation of Labeled Fragment:

Step A: Horseradish peroxidase (100 mg dry weight) was dissolved in sodium carbonate (13.4 ml, 0.1 molar pH 9.5) at 4°C and reacted with a solution of S-acetylmercaptosuccinic anhydride in dry N,N-dimethylformamide (300 μl at 17.4 mg/ml) for one hour at 4°C or lower. This mixture was transferred by pipette into a SpectroPorTM dialysis bag (available from Spectrum Medical Ind., Los Angeles) that had been prewet with deionized distilled water for 10 minutes. The bag was then placed into phosphate buffered saline solution (pH 7.4) using 50 times the volume of the reaction mixture, and slowly stirred at 4°C for about four hours. The solution volume was concentrated using an Amicon concentrator to give the desired intermediate (18.6 mg/ml).

The intermediate just prepared (2.61 ml of solution containing 18.6 mg/ml) in phosphate buffered saline solution (pH 7.4) was unblocked by reaction with a solution containing hydroxylamine (0.652 ml, 0.25 molar) in phosphate buffer (0.25 molar, pH 7.5) and ethylenediaminetetraacetic acid (0.001 molar) for two hours at room temperature. The resulting enzyme reagent was purified by chromatography using a PD-10 column and phosphate buffered saline solution (pH 7.4) as the eluent.

Step B: The purified $F(ab')_2$ fragment described above (13.85 mg of a solution of 2.5 mg/ml in phosphate buffered saline solution) was mixed with succinimidyl-4-(N-maleimidomethyl)cyclohexane-1-carboxylate (130.2 μl of 16.7 mg/ml N,N-dimethylformamide) and shaken for two hours at 4°C. The resulting product was purified by chromatography using a PD-10 column and phosphate buffered saline solution (pH 7.4) as the eluent.

Step C: The enzyme reagent (2.6 ml of a solution of 18.6 mg/ml of phosphate buffered saline solution) prepared in Step A above, and the product (10.5 ml of a solution of 1.32 mg/ml of phosphate buffered saline solution) prepared in Step B were mixed, the concentration was adjusted to about 1 mg/ml of the $F(ab')_2$ fragment, and then the mixture was rotated for about 15 hours at 25°C. The resulting conjugate of enzyme-labeled fragment was dialyzed against 2-(N-morpholino)ethanesulfonic acid buffer (40 μl, 25 mmolar, pH 5.6) using an Amicon concentrator with a 10,000 molecular weight cutoff membrane. The resulting product (5 ml) was then purified using an ABx column, eluting with 15 ml of a solution of 2-(N-morpholino)ethanesulfonic acid, followed by a gradient solution (about 30 ml) of ammonium sulfate (0.5 molar) and sodium acetate (20 mMolar), pH 6.7. Fractions having an absorbance ratio (403/280) of about 0.6 were pooled to give 4.85 mg of the desired peroxidase labeled fragment.

The labeled fragment was stored in a phosphate buffered saline solution (0.2% solution, pH 7.4) containing merthiolate preservative (0.01%) at 4°C. The molecular weight range of the labeled fragment was about 135 to about 250 kdaltons.

Example 2: Assay for Chlamydia Trachomatis

This example illustrates the practice of the present invention and compares it to an assay wherein an enzyme labeled whole antibody is used. In addition, the invention is compared to an assay wherein two antibodies are used, that is, an unlabeled anti-chlamydial antibody, and an enzyme-labeled anti-antibody specific to the anti-chlamydial antibody.

Materials Used:

The following materials and compositions were used in the assay.

The microporous membrane used in the practice of this invention was an uncharged and uncoated nylon 66 membrane, a LoProdyneTM microporous membrane (Pall Corp.). This membrane was mounted in each of the three test wells of disposable test devices.

Specimens for testing were obtained from female patients using endocervical swabs. Each specimen was tested using separate test devices having the Control membrane or the surfactant-coated membrane of the present invention.

An extraction tube was used to extract chlamydial antigen from the specimens, the tube having at separate locations on the inside thereof, dried coatings of: (1) tris(hydroxymethyl)aminomethane buffer (20 $\mu$l of a 1.65 molar solution, pH 11) with thimerosal preservative (0.01%), and (2) 2-(N-morpholino)ethane sulfonic acid (10 mmolar, 50 $\mu$l solution, pH 6), sodium azide (1.54 mmolar), ethylenediaminetetraacetic acid (5.4 mmolar), 5,5-dimethyl-1,3-cyclohexanedione (21.4 mmolar), dithiothreitol (0.188 molar) and poly-(acrylamide)(6.35%).

Composition 1 contained AmideckTM protease (4 mg/ml, 170 units/mg, available from Genencor, International, Rochester, N.Y.) in 10 mMolar 2-(N-morpholino)ethane sulfonic acid buffer (pH 6), sodium chloride (50 mMolar), calcium chloride (5 mmolar), 1,2-propanediol (10% w/v) and thimerosal (0.01%).

An extraction composition contained ethanolamine hydrochloride (0.47 molar), sodium chloride (0.27 molar), thimerosal (30 mmolar) ethylenediaminetetraacetic acid (50 mmolar), EmcolTM CC-36 cationic surfactant (0.45% from Witco Chemical) and sodium hydroxide (0.66 normal, to provide a pH of 13.5).

Composition 2 contained hydrogen peroxide (12% in water), diethylenetriaminepentaacetic acid (10 $\mu$molar) and thimerosal (0.01%).

A wash solution contained 3-cyclohexylamino-2-hydroxy-1-propanesulfonic acid buffer (0.05 molar, pH 10), EmcolTM CC-9 cationic surfactant (0.75%) and thimerosal (0.01%).

Composition 3 contained creatine kinase-MB F(ab')$_2$ antibody fragment conjugated to horseradish peroxidase (5 $\mu$g/ml), casein (0.01%), LonzaineTM C amphoteric surfactant (0.01% from Lonza Corp.) and thimerosal (0.01%) in phosphate buffered saline solution (pH 7.2). This composition was used as in a test well of each test device as a negative control. The labeled fragment was prepared by the same procedure as described in Example 1 above for the anti-chlamydial fragment. However, the final chromatography was done using a standard DEAE column rather than a standard ABx column. Eluting buffers used were: (1) tris-(hydroxymethyl)aminomethane (20 mmolar, pH 8), and (2) tris(hydroxymethyl)aminomethane (20 mmolar, pH 8) containing sodium chloride (0.75 molar).

The labeled fragment prepared in Example 1 above (4 $\mu$g/ml of solution, final concentration) was mixed in solution with casein (0.05%), LonzaineTM C amphoteric surfactant (0.01%, Lonza Corp.) and thimerosal (0.01%) in phosphate buffered saline solution (pH 7.2).

A dye-providing composition comprised 2-(4-hydroxy-3-methoxyphenyl)-4,5-bis(4-methoxyphenyl)-imidazole leuco dye (0.008%), poly(vinyl pyrrolidone) (1%), sodium phosphate (10 mmolar, pH 6.8) diethylenetriaminepentaacetic acid (10 $\mu$molar), 4'-hydroxyacetanilide (2 mmolar) and hydrogen peroxide (10 mmolar).

Assay:

In assaying each specimen, Composition 1 (7 drops) was added to the extraction device and a patient swab containing each specimen was placed into the device, rotated 10 seconds, followed by incubation of the device for 1 minute at room temperature (18-25° C). The extraction composition was then added to the device containing the swab which was rotated again for 10 seconds, and incubation was again carried out for 1 minute at room temperature. Composition 2 was then added, and the swab was rotated again for 10 seconds, followed by a third incubation for two minutes at room temperature. The swab was wrung out by squeezing the tube, and removed therefrom. The extraction procedure required about 4 to 5 minutes.

The resulting solution containing extracted lipopolysaccharide antigen was removed from the device using a pipette, prefiltered, and transferred to each well of a disposable test device (4 drops per well). Fluid was allowed to drain through the microporous membranes in the wells. Each well was then washed with the wash solution (4 drops per well).

Composition 3 (2 drops) was then added to one of the wells of each test device (considered a negative control well). The immunological composition of this invention (2 drops) was added to each of the two remaining wells of each device. One of those two wells (considered a positive control well) contained dried chlamydial lipopolysaccharide antigen on the membrane.

After incubation for two minutes at room temperature, the wash step was repeated twice. The dye-providing composition was added and after three minutes incubation at room temperature, the test sample well in each test device showed a highly visible dye on its membrane, indicating positive detection of C. trachomatis.

The assay from the time the extract was added to the test devices required less than about five minutes, which is considerably less than the twelve minutes generally required for the assay described in EP-A-0 363 090. Even with the extraction time included, the entire assay required less than about 9 minutes which is very rapid compared to either the EP-A-0 363 090 assay or the assay of US-A-4,497,899.

Example 3: Comparative Stability Studies

This example compares the stability of the enzyme-labeled fragment used in this invention with the stability of an enzyme-labeled whole antibody.

Materials:

The peroxidase-labeled F(ab')$_2$ fragment prepared as described in Example 1 above was diluted (1 $\mu$g/ml) in Medix General Conjugate Diluent (Medix Biotech Inc., Foster City, California) and incubated for 49 days at 37° C.

The peroxidase-labeled whole antibody directed to the lipopolysaccharide antigen of C. trachomatis was prepared using the method of Yoshitake and others, Eur.J.Biochem., 101, 395 (1979), and diluted in the Medix General Conjugate Diluent as noted above, and incubated for 47 days at 37° C.

Chlamydial antigen solution comprised chlamydial serovar G elementary bodies in phosphate buffered saline solution (pH 7.2) and contained bovine serum albumin (0.1 mg/ml).

An interferent background composition contained whole blood (25 $\mu$l) pig mucin (Sigma, 20 $\mu$l of 125 mg/ml phosphate buffered saline solution), HL60 cells (5 $\mu$l, 5 x 10$^7$ cells/ml of phosphate buffered saline solution, pH 7.2). The total volume was 50 $\mu$l. This composition was prefiltered before use.

A blank background solution contained bovine serum albumin (0.1 mg/ml) in phosphate buffered saline solution (pH 7.2).

The test devices, extraction devices and other compositions and solutions were the same as those described in Example 2 above. However, Composition 2 contained 20% hydrogen peroxide instead of 12% as in Example 2.

Assay:

Two similar assay procedures were carried out using the chlamydial antigen solution, the interferent background composition and the blank background solution. One procedure was carried out with the peroxidase-labeled F(ab')$_2$ reagent (Invention), and the other was carried out using the peroxidase-labeled whole antibody (Control). Duplicate tests were made with each solution in separate Surecell™ test devices.

Invention Test:

The elementary bodies were added to an extraction device, followed by the addition of 7 drops (about 280 $\mu$l) of Composition 1, and the device was incubated at room temperature for one minute The extraction solution (280 $\mu$l) was added to the device, followed by incubation at room temperature for another minute. Composition 2 (7 drops) was then added and the device was incubated at room temperature for one minute. The resulting extract solution was removed from the device with a pipette and a sample (160 $\mu$l, containing 500 pg of antigen) was added to each of two duplicate test wells in a Surecell™ test device.

After the sample had drained through the membrane in the test device, the wells were washed twice with wash solution (250 $\mu$l each). The peroxidase-labeled F(ab')$_2$ fragment solution (1 drop) was added to both test wells, allowed to drain, then the device was incubated at room temperature for two minutes. The test wells were again washed twice (250 $\mu$l each), and allowed to drain. The dye-providing composition (2 drops) was added to the test wells, and after three minutes incubation at room temperature, the dye on the membrane was visually read and scored against a graduated color chart with values ranging from 0 to 10, with 0 representing no visible dye and 10 representing the highest dye density.

The interferent background composition and the blank background solution were extracted as described above for the test samples, added to duplicate test wells of separate test devices (160 $\mu$l to each well), and assayed as described above. The results are shown in the following Table as the averages of the duplicate tests.

Control Test:

Second portions (160 μl each) of extracted antigen, interferent background and blank background solutions were added to duplicate test wells of separate Surecell™ test devices as above, using the peroxidase-labeled whole antibody (1 drop), and assayed as described above. The results of the assays are shown below in the Table as the averages of the duplicate tests.

## T A B L E

### Visual Dye Densities

| | Invention Test | Control Test |
|---|---|---|
| Chlamydial Elementary Bodies Sample | 4.8 | 5.0 |
| Interferent Background Composition | 2.2 | 3.0 |
| Blank Background Solution | 0.5 | 1.3 |

The results shown in this table demonstrate that although the assays using the chlamydial elementary bodies are similar, the background densities for the Control test are considerably higher than the background for the Invention test. This indicates that the labeled fragment is more stable over time than the labeled whole antibody.

Example 4: Use of Buffered Compositions in Assay for Chlamydia Trachomatis

This example has been taken from Examples 1-5 of EP Application Serial No. _____ corresponding to USSN 522,441 filed 11 May 1990), except that irrelevant comparisons have been omitted. This example demonstrates the use of an antibody fragment in a preferred composition with a heme-containing protein.

Materials:

Chlamydial antigen solution comprised chlamydial Serovar G elementary bodies in phosphate buffered saline solution (pH 7.2) containing bovine serum albumin (0.1 mg/ml). A Blank Control solution comprised bovine serum albumin (0.1 mg/ml) in phosphate buffered saline solution (pH 7.2) without antigen.

Surecell™ disposable test devices (Eastman Kodak Co.) were used containing uncoated LoProdyne™ microporous membranes (5 μm, Pall Corp.) in each test well.

An extraction tube was used to extract chlamydial antigen from the elementary bodies having at separate locations on the inside thereof, dried coatings of: (1) tris(hydroxymethyl)aminomethane buffer (Sigma Chemical, 20 μl of a 1.65 molar solution, pH 11) with thimerosal preservative (0.01%), and (2) 2-(N-morpholino)ethane sulfonic acid (10 mmolar, 50 μl solution, pH 6), sodium azide (1.54 mmolar), ethylenediaminetetraacetic acid (5.4 mmolar), 5,5-dimethyl-1,3-cyclohexanedione (21.4 mmolar), dithiothreitol (188 mmolar) and poly(acrylamide)(6.35%).

Composition 1 contained Amideck™ protease (4 mg/ml, 170 units/mg, available from Genencor, International, Rochester, N.Y.) in 10 mmolar 2-(N-morpholino)ethane sulfonic acid buffer (pH 6), sodium chloride (50 mmolar), calcium chloride (5 mmolar), 1,2-propanediol (10% w/v) and thimerosal (0.01%).

An extraction composition contained ethanolamine hydrochloride (0.47 molar), sodium chloride (0.27 molar), thimerosal (30 mmolar) ethylenediaminetetraacetic acid (50 mmolar), Emcol™ CC-36 cationic surfactant (0.45% from Witco Chemical) and sodium hydroxide (0.66 normal, to provide a pH of 13.5).

Composition 2 contained hydrogen peroxide (12% in water), diethylenetriaminepentaacetic acid (10 μmolar) and thimerosal (0.01%).

A wash solution contained 3-cyclohexylamino-2-hydroxy-1-propanesulfonic acid buffer (0.05 molar, pH

10), EmcolTM CC-9 cationic surfactant (0.75%) and thimerosal (0.01%).

A dye-providing composition comprised 2-(4-hydroxy-3-methoxyphenyl)-4,5-bis(4-methoxyphenyl)-imidazole leuco dye (0.008%), poly(vinyl pyrrolidone) (1%), sodium phosphate (10 mmolar, pH 6.8) diethylenetriaminepentaacetic acid (10 $\mu$molar), 4'-hydroxyacetanilide (2 mmolar) and hydrogen peroxide (10 mmolar).

Five buffered compositions (identified herein as Compositions I-V) were prepared by mixing the labeled fragment prepared as described above (4 $\mu$g/ml of solution, final concentration) with a heme-containing protein (0.11 to 1.25 mg/ml, as noted below), casein (0.05%), LonzaineTM C amphoteric surfactant (0.01%, Lonza Corp.) and thimerosal (0.01%) in phosphate buffered saline solution (pH 7.2). None of these compositions included 4'-hydroxyacetanilide, which is a phenolic electron transfer agent.

Compositions I, II, III, IV and V contained the following amounts of heme-containing protein:

Composition I: 0.125 mg/ml of myoglobin.
Composition II: 0.25 mg/ml of myoglobin.
Composition III: 1.25 mg/ml of myoglobin.
Composition IV: 0.11 mg/ml of cytochrome c.
Composition V: 0.23 mg/ml of cytochrome c.

Assay Procedure:

For each of Compositions I-V, the following assay procedure was followed.

Composition 1 (8 drops) was added to an extraction tube, followed by the elementary bodies, and mixed for 5-10 seconds. The tube and contents were then incubated for 1 minute at room temperature (18-25°C). The extraction composition (8 drops) was added and mixed 5-10 seconds with the tube's contents, then incubated one minute at room temperature. Composition 2 (8 drops) was added, mixed 5-10 seconds, and incubated with the tube's contents for two minutes at room temperature.

The resulting solution containing extracted lipopolysaccharide antigen was removed from the tube using a pipette, prefiltered, and transferred to each well of a SurecellTM disposable test device (160 $\mu$l containing 500 pg of antigen per well). Fluid was allowed to drain through the microporous membranes in the wells. Each well was then washed twice with the wash solution (250 $\mu$l each), and allowed to drain.

Each of Compositions I-V (1 drop each) was then added to test wells (of devices to which antigen had been added), followed by incubation at room temperature for two minutes to form an immunological complex on the membrane of each well. The wells were washed three times (250 $\mu$l each) and allowed to drain each time.

The dye-providing composition (2 drops) was added and after three minutes incubation at room temperature, the dye formed on the membranes was evaluated against a graduated color chart having values of 0 to 10, with 0 representing no dye density and 10 representing the highest dye density. Each of the tests using Compositions I-V showed an acceptable dye density signal from the presence of antigen in the test well.

**Claims**

1. A method for the determination of a chlamydial or gonococcal antigen comprising;
    A. contacting chlamydial or gonococcal antigen from a specimen suspected of containing chlamydial or gonococcal organisms, respectively, with a microporous membrane which is substantially free of any biological compound reactive with the antigen so as to bind the antigen directly to the membrane,
    B. within 5 minutes of the contact in step A, contacting the bound antigen with an immunological composition comprising: an enzyme-labeled F(ab')$_2$ fragment immunologically directed to the chlamydial or gonococcal antigen, respectively,
        so as to form a labeled immunological complex bound to the membrane, and
    C. within 10 minutes of the contact in step B, determining the presence or amount of the bound labeled complex on the membrane as a measure of the presence or amount of chlamydial or gonococcal organisms, respectively, in the specimen.

2. The method as claimed in claim 1 wherein the complex determination is accomplished by contacting the complex with a dye-providing composition which comprises a substrate for the enzyme label.

3. The method as claimed in either of claims 1 and 2 which is carried out using a disposable test device in which the microporous membrane is mounted.

4. The method as claimed in any of claims 1 to 3 for the determination of a chlamydial antigen.

5. A method for the determination of a chlamydial antigen comprising:
   A. extracting chlamydial antigen from chlamydial cells present in a biological specimen,
   B. contacting the extracted chlamydial antigen with a polyamide microporous membrane which is substantially free of any biological compound reactive with the antigen so as to bind the antigen directly to the membrane,
   C. within 2 minutes of the contact in step A, separating unbound materials from the bound antigen, and contacting the bound antigen with an immunological composition comprising: an enzyme-labeled $F(ab')_2$ fragment immunologically directed to the chlamydial antigen,
      so as to form a labeled immunological complex bound to the membrane, and
   D. separating uncomplexed materials from the bound labeled complex by washing, and
   E. contacting the complex with a dye-providing composition which comprises a substrate for the enzyme label thereby determining the presence or amount of the bound labeled complex on the membrane as a measure of the presence or amount of chlamydial organisms in the specimen,
      the method from steps B to E being carried out in less than ten minutes.

6. The method as claimed in any of claims 1 to 5 for the determination of a lipopolysaccharide.

7. A buffered immunological composition comprising an enzyme-labeled immunoreactant, the composition characterized wherein the immunoreactant is a $F(ab')_2$ fragment directed to either a chlamydial or gonococcal organism or to an antigenic component thereof.

8. The use of a buffered immunological composition comprising an enzyme-labeled $F(ab')_2$ fragment directed to either a chlamydial or gonococcal organism or to an antigenic component thereof.

9. A diagnostic test kit useful for the determination of chlamydial or gonococcal organisms comprising:
   A. a buffered immunological composition comprising an enzyme-labeled immunoreactant, and
   B. a dye-providing composition comprising a substrate for the enzyme label,
      the test kit characterized wherein the immunoreactant is a $F(ab')_2$ fragment directed to either a chlamydial or gonococcal organism or to an antigenic component thereof.

10. A method for the determination of a chlamydial or gonococcal antigen comprising: detecting the presence or amount of either an uncomplexed enyzme-labeled $F(ab')_2$ fragment directed to a chlamydial or gonococcal antigen or an enzyme-labeled complex of the enzyme-labeled fragment formed with the antigen, after the antigen is contacted with a buffered composition comprising the enzyme-labeled fragment, and after uncomplexed fragment is separated from any complex formed between the antigen and enzyme-labeled fragment.

11. An enzyme immunoassay employing $F(ab')_2$ antibody fragments conjugated with an enzyme to form enzyme conjugates for detecting the presence or amount of a chlamydial or gonococcal organism in a specimen,
      the immunoassay comprising addition of a buffered composition comprising a conjugate of an enzyme and an $F(ab')_2$ fragment which is specifically reactive with a chlamydial or gonococcal organism, or with an antigenic component thereof.

12. The invention as claimed in any of claims 1 to 11 wherein the immunological composition comprises a plurality of enzyme-labeled $F(ab')_2$ fragments, each immunologically specific to an antigen of a specific chlamydial or gonococcal strain.

13. The invention as claimed in any of claims 1 to 12 wherein the immunological composition further comprises a nonimmunological protein and an amphoteric surfactant.

European
Patent Office

**EUROPEAN SEARCH
REPORT**

Application Number

**EP 91 20 1042**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 363 090 (EASTMAN KODAK CO.) <br> * Whole document * <br> – – – | 1-13 | G 01 N 33/571 <br> G 01 N 33/563 <br> G 01 N 33/569 |
| Y | EP-A-0 345 462 (ABBOTT LABORATORIES) <br> * Whole document * <br> – – – | 1-13 | |
| A,D | US-A-4 397 960 (C.H. MOUSSEBOIS et al.) <br> * Whole document * <br> – – – – – | 1-13 | |

| TECHNICAL FIELDS SEARCHED (Int. Cl.5) |
|---|
| G 01 N |

The present search report has been drawn up for all claims

| Place of search | Date of completion of search | Examiner |
|---|---|---|
| The Hague | 09 August 91 | VAN BOHEMEN C.G. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document